(19) Europäisches Patentamt
European Patent Office
Office européen des brevets

(11) Publication number: 0 231 077
A2

(12) # EUROPEAN PATENT APPLICATION

(21) Application number: 87300312.3

(22) Date of filing: 15.01.87

(51) Int. Cl.[3]: C 07 K 7/06
C 07 K 7/16, A 61 K 37/02

(30) Priority: 16.01.86 US 819336

(43) Date of publication of application:
05.08.87 Bulletin 87/32

(84) Designated Contracting States:
AT BE CH DE ES FR GB GR IT LI LU NL SE

(71) Applicant: SMITHKLINE BECKMAN CORPORATION
P.O. Box 7929 1 Franklin Plaza
Philadelphia Pennsylvania 19101(US)

(72) Inventor: Callahan, James Francis
5744 Rising Sun Avenue
Philadelphia Pennsylvania 19120(US)

(72) Inventor: Huffmannn, William Francis
40 Crest Avenue
Malvern Pennsylvania 19355(US)

(72) Inventor: Yim, Nelson Chi-Fai
669 Meadowbrook Avenue
Ambler Pennsylvania 19002(US)

(74) Representative: Waters, David Martin, Dr. et al,
Smith Kline & French Laboratories Ltd. Patent
Department Mundells
Welwyn Garden City Hertfordshire AL7 1EY(GB)

(54) **Polypeptide compounds.**

(57) New compounds which have potent $V_2$-vasopressin antagonistic activity are prepared by a 1,6-cyclization using peptide bond formation. The structures of the compounds are characterized by a $Pas^{1,6}$ or $Tas^{1,6}$ cyclized unit.

EP 0 231 077 A2

## POLYPEPTIDE COMPOUNDS

This invention relates to new vasopressin compounds whose structures are distinguished by having a 1,6-dicarba bridge which is combined with a spirocycloalkyl substituent at the 1-position. The compounds have potent in vivo and in vitro vasopressin antagonist activity.

Examples of the dithia or vasopressin antagonist art are U.S. Patent Nos. 4,469,679, 4,481,193, 4,481,194 and 4,491,577.

Carba analogues of oxytocin and vasopressin, which have agonist activity, have been repeatedly reported in the prior art, such as in U.S. Patent Nos. 3,980,631, 4,285,858, 4,482,486, 4,237,119 as well as in F. Fahrenholz et al., Biochem. Biophys. Res. Com. 122, 974 (1984) and J. Biol. Chem. 258 14861 (1983). Dicarba or 1,6-aminosuberic acid analogs of lysine- and arginine-vasopressin and oxytocin have been reported to have variable agonist activity. These prior art structures have no substituent on the 1,6-suberic acid and are agonists, i.e. they have the same biological activity as do vasopressin (VSP) or oxytocin (OXT).

We have now found that, when the 1-unit and the dithia connection of certain vasopressin structures is replaced by a 6,6-spiroalkylenesuberic acid, the resulting compounds have potent, even enhanced antagonist activity, especially aquaretic activity.

In the description herein and in the claims, the nomenclature common in the art of peptide and vasopressin chemistry is used. When no configuration is noted, the amino acid unit is in the L, or naturally occurring, form.

Certain of the peptide art designations used herein are the following: Pas, 6,6-cyclopentamethylene-2-aminosuberic acid; Tas, 6,6-cyclotetramethylene-2-aminosuberic acid; Pas(Bzl), w-benzyl ester of Pas; Abu, α-amino-n-butyric acid; Cad, cadaverine, $-NH-(CH_2)5-NH_2$; Chg, cyclo-hexylglycine; Cha, cyclohexylalanine; Thr, threonine; Pba, α-aminophenylbutyric acid; Gln, glutamic acid amide or glutamine; Pro, proline, ΔPro, $\Delta^3$-proline; Gly, glycine; Tyr, tyrosine; Tyr(Alk), lower alkyl ether of Tyr; Phe, phenylalanine; Phe(4'-Alk), 4'-alkylphenylalanine; MeAla, N-methylalanine; Val, valine; Ile, isoleucine; Nle, norleucine; Leu, leucine; Ala, alanine; Lys, lysine; Arg, arginine; HArg, homoarginine; MeArg, N-methylarginine; MeHArg, N-methylhomoarginine; MeLys, N-methyllysine; Met, methionine; Asn, asparagine; Tos, tosylate; BHA, benzhydrylamine; DMAP, 4-dimethylaminopyridine; DIEA, diisopropylethylamine; Trp, tryptophan; HF, hydrogen fluoride; 4-MeBzl, 4-methylbenzyl; TFA, trifluoroacetic acid; DCC, dicyclohexylcarbodiimide; Boc, t-butyloxycarbonyl; Z, benzyloxycarbonyl; VSP, vasopressin; HBT, hydroxybenzotriazole. In the definitions such as MeArg above, Me denotes a methyl located on the amido nitrogen of the peptide unit concerned. The designation, $Pas^{1,6}$, is used to denote the cyclized peptide as described hereafter.

"Alk" represents a lower alkyl of 1-4 carbons. For example, these may be optionally attached to the oxygen substituent of a tyrosine unit at position 2 of the peptide of formula I, to the N-terminal nitrogen of the tail, or to the 4'-position of a Phe unit at position 2 or 3 of the peptide of formula I. Such alkyl substituents include methyl, ethyl, n-propyl, isopropyl or butyl. Ethyl is preferred. When the term "vasopressin" is used, it means L-arginine vasopressin (AVP) unless otherwise modified.

The dicarba-vasopressin compounds of this invention are illustrated by the following structural formula:

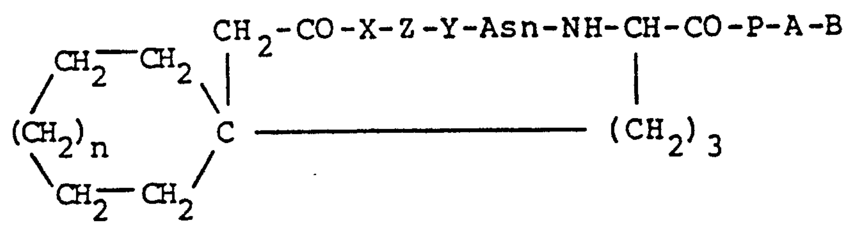

I

in which:

n is 0 or 1;

P is a single bond or a D or L isomer of Pro, Δ-Pro, Ala, MeAla, Arg, Lys, HArg, MeArg, MeLys or MeHArg;

A is a D or L isomer of Arg, Lys, HArg, MeArg, MeLys, MeHArg or, when B is $-NH-(CH_2)n^1-NH_2$, a single bond;

B is OH, $NH_2$, NHAlk, Gly, Gly($NH_2$), Gly(NHAlk) or, when A is a single bond, $-NH-(CH_2)n^1-NH_2$;

Z is Phe, Phe(4'-Alk), Tyr(Alk), Ile or Tyr;

X is a D or L isomer of Phe, Phe(4'-Alk), Val, Nva, Leu, Ile, Pba, Nle, Cha, Abu, Met, Chg, Tyr, Trp or Tyr(Alk); and

Y is Val, Ile, Abu, Ala, Chg, Gln, Lys, Cha, Nle, Thr, Phe, Leu or Gly; and

$n^1$ is 2 to 6.

A subgeneric group of compounds of this invention comprises compounds of formula I in which P is a single bond, Pro or Arg, A is Arg and B is $NH_2$. In formula I, n is preferably 1 and X is a D-isomer unit.

Also included in this invention are addition salts, complexes or prodrugs, such as esters of the compounds of this invention when B is OH, especially the nontoxic, pharmaceutically acceptable acid addition salts. The acid addition salts are prepared in standard manner in a suitable solvent from the parent compound and an excess of an acid, such as hydrochloric, hydrobromic, sulfuric, phosphoric, acetic, maleic, succinic, ethanedisulfonic or methanesulfonic acids. Certain end products of formula I such as the $Arg^7$-$Arg^8$ compounds have two strong basic groups in their structures, therefore, their acid addition salt derivatives are easily prepared. The ester derivatives of the acid forms of the end products, such as the methyl, ethyl or benzyl esters, are prepared as known to the art.

The end products (I) of this invention are prepared by cyclization of the corresponding linear peptide:

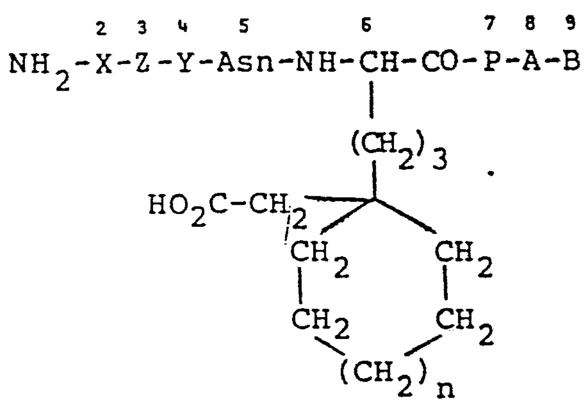

II

in which X, Z, Y, P, A, B and n are as defined above with any chemically reactive centers protected as described below. Also included are any addition salt forms as noted above for the end compounds of formula I. It should be emphasized that the linear intermediate structure II can be cyclized only in the form of the free base. No cyclization occurs when the salt form is present but the salt forms are important derivatives for isolation and characterization of the intermediates.

The cyclization involves the formation of an amide bond between the α-amino group of the X unit at position 2 and the free ω-carboxy group of the 6,6-substituted 2-amino suberic acid unit at position 6 of structure II. Any chemical method of amide formation is employed such as reaction using dicyclohexylcarbodiimide plus hydroxybenzotriazole or 4-dimethylaminopyridine, reaction using diphenylphosphoryl azide (DPPA) and base, 1-(3-dimethylaminopropyl)-3-ethylcarbodiimide (EDC) as the methiodide or hydrochloride; the azide derivative of the carboxylic acid; a mixed anhydride such as the benzoyl, pivaloyl, or isovaleroyl containing anhydride with the suberic acid; ethoxyacetylene or N-ethyl-5-phenyl-isoxazolium-3'-sulfonate. All these general synthetic methods are described in Peptide Synthesis, M. Bodanszky, John Wiley, 1976, page 191 and Table 3, pages 116-121. These coupling reactions are usually run in either aqueous or organic solvents until reaction is complete. Among the preferred solvents are dimethyl formamide, dimethylacetamide, methylene chloride, acetonitrile or combinations thereof. The reactions are usually carried out at room temperature and in dilute solution to minimize linear reaction.

Diphenylphosphoryl azide (DPPA) is a particularly useful cyclization agent. The linear peptide (II) is treated with an excess of DPPA in dried dimethylformamide and triethylamine at room temperature until thin layer

monitoring demonstrates completion of the reaction. The desired product is isolated by evaporation and purified by gel filtration and high performance liquid chromatography.

The resin supported peptide chain of the linear peptides (II) is usually built up, stepwise, proceeding from the B unit and working toward the X unit through the novel Pas or Tas unit at position 6 of the linear product. Each unit is properly protected as known in the peptide art and as described below. The sequence of step-reactions is conveniently carried out in a Beckman 990B peptide synthesizer or its equivalent without isolation of each intermediate peptide. The details of the procedure are in the working examples presented hereinafter.

The various amino acids (AA), which are consecutively added to the resin-supported chain, are protected as known to the art. For example, the Boc protecting group is used for an amino group, especially at the α-position; an optionally substituted benzyl for the carboxy group of the Pas or Tas unit; tosyl for the Arg, HArg or MeArg unit; and an optionally substituted carbobenzyloxy (Z) for the Tyr or Lys units. The protective groups are, most conveniently, those which are not easily removed by using mild acid treatment, such as for removing the Boc group. Rather one should use HF, sodium-liquid ammonia or, for benzyl or carbobenzyloxy groups, catalytic hydrogenation.

Often, the uncyclized, resin-supported polypeptide of Formula II is synthesized in a number of synthetic cycles to offer good supplies of oligopeptide intermediates to vary the chain as described above.

The assembled, resin-supported peptide is treated with an excess of anhydrous hydrogen fluoride with an appropriate scavenger compound, such as anisole, to give the linear peptide intermediate of formula II in good yield.

The compounds of formula I are also prepared by reacting the cyclized $Pas^6$, $Pro^7$ or the $Arg^{7 \text{ or } 8}$ carboxylic acids or derivatives thereof with a protected form of PAB, AB or A in the terminal acid ester or amide form, respectively, employing reaction conditions and intermediates of standard peptide methods of synthesis. Such starting material acids, such as those of formula I in which P is an arginine-like unit as defined above and A is hydroxy, are prepared as described above by either a resin-supported or a solution reaction sequence.

The key to the synthesis of the compounds of formula I and the discovery of their biological activity was the availability of the 6,6-spirocycloalkylene-2-aminosuberic acid which must be available for use in the peptide synthesis of the intermediates of formula II discussed above. 2-Amino and 2,7-diaminosuberic acids had been prepared earlier using a Kolbe electrolysis as the key step of the synthesis, R. Nutt et al., J. Org. Chem. 45 3078 (1980). The presence of the bulky spirocycloalkylene substituent at the 6,6-position of the desired 2-aminosuberic acid made the preparation of these intermediates and the cyclization of the linear peptides of formula II unpredictable prior to the present invention.

The synthetic preparation of 6,6-spirocycloalkylene-2-aminosuberic acids as well as the corresponding 7,7-substituted azelaic and 5,5-substituted pimelic acids involves (1) the insertion of a terminally unsaturated hydrocarbon chain at the spiro carbon of a cycloalkane carboxylate, (2) homologizing the carboxylate, (3) functionalizing the unsaturated center of the side chain and (4) preparation of the amino acid therefrom. Details of the preparation are presented in Example 1.

The spirocycloalkylene-2-aminoalkandioic acid intermediates are represented by the following formula:

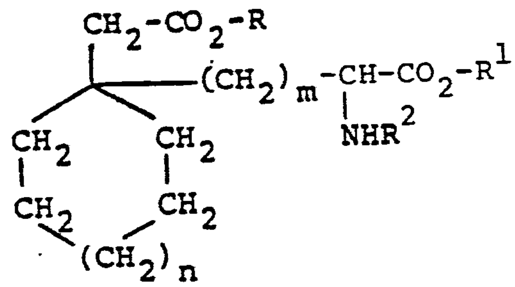

III

in which:

m is 2-4;

n is 0 or 1;

R and $R^1$ are, each, hydrogen or a carboxy protecting group; and

$R^2$ is hydrogen or an amino protecting group.

Carboxy protecting groups, which are useful at R or $R^1$ of formula III, are well known in the peptide art, Peptide Synthesis, loc. cit. 49-57. Representative groups are lower alkyl of 1-8 carbons, benzyls, benzhydryls, phenyls all which form ester derivatives but which are easily removed by methods known to the art. Preferably, selective removal methods must be available after the linear peptide of formula II has been prepared.

Amino protective groups, which are useful in formula III at $R^2$ are also well known, Peptide Synthesis, loc. cit. 18-48. Particularly useful are carbobenzoxy, t-butyloxycarbonyl, p-toluene sulfonyl, trifluoroacetyl, arylsulfenyl or formyl groups.

Also included in formula III are the optically active isomers. These are separated from isomeric mixtures into chirally pure D- and L-isomeric form by fractional crystallization of salts with chiral bases.

The end compounds of the invention have vasopressin antagonist activity. Vasopressin is known to contribute to the anti-diuretic mechanism of action within the kidney. When the action of these compounds antagonizes that of the natural anti-diuretic hormone

(ADH), the body excretes water due to an increased permeability of the terminal portions of the renal tubule. The mechanism of action is at the vasopressin receptors ($V_2$-receptors) located on the plasma membrane of certain renal epithelial cells. The most notable pharmacodynamic effect of the ADH antagonists of the invention is that of a water diuretic rather than of a natriuretic such as hydrochlorothiazide.

Any patient suffering from the syndrome of inappropriate antidiuretic hormone secretion (SIADH) or from an undesirable edematous condition is a target for the claimed compounds. Examples of clinical conditions indicated for the compounds of this invention include hypertension, hepatic cirrhosis, hyponatremia, congestive heart failure or a component of any traumatic condition resulting from serious injury or disease.

The second group of vasopressin receptor sites are the vascular pressor sites ($V_1$-receptors) within the cardiovascular system itself. These are also somewhat antagonized by the compounds of this invention thereby inducing antipressor or hypotensive activity.

The compounds of this invention, therefore, are used especially to induce anti-hypertensive or diuretic activity in patients in need of such treatment. This comprises the administration internally, parenterally, buccally or by insufflation, of a nontoxic but effective quantity of the chosen compound, preferably dispersed in a pharmaceutical carrier. Dosage units of the active ingredient are selected from the range of 0.01 to 10 mg/kg, preferably 0.1 to 1 mg/kg, of base based on a 70 kg patient. The dosage units are administered to the human or animal patient from 1 to 5 times daily.

The pharmaceutical composition, which contains an active antagonist ingredient of formula I, comprises a dosage unit which is dissolved or suspended in a standard liquid carrier, such as isotonic saline, and is contained

in an ampoule or a multiple dose vial suitable for a parenteral injection such as for intravenous, subcutaneous or intramuscular administration. A composition for insufflation may be similar but is usually administered in a metered dose applicator or inhaler. Pulverized powder compositions may, also, be used along with oily preparation, gels, buffers for isotonic preparations, buccal losenges, trans-dermal patches and emulsions or aerosols.

$V_2$-antagonistic activity toward the natural anti-diuretic hormone (anti-ADH activity) is determined, in vitro, in the medullary tissue of hog ($K_b$ in Table I below) or human kidneys and, in vivo, in the hydropenic rat. The in vitro assay procedures for vasopressin stimulated adenylate cyclase activation (Ki) or vasopressin binding activity Kbind are described by F. Stassen et al., J. Pharmacology and Experimental Therapeutics, 223, 50-54 (1982). $V_1$-antagonistic activity is determined by procedures using the rat thoracic aorta tissue and plasma membranes of rat liver. These procedures are described in the noted Stassen publication and in a publication at the 1st International Conference on Diuretics, Miami, Florida, March (1984). Oxytocin antagonism is determined as described by W. Sawyer et al., Endocrinology, 106 81 (1979).

The assay for anti-ADH activity in vivo is the hydropenic rat protocol described below:

Hydropenic Rat Screen

Food and water are removed from male rats approximately 18 hours prior to testing. Animals are housed 4 per metabolism cage. At 0 hour, the test compound is administered intraperitoneally to the test group and an equivalent volume of vehicle is administered to both control groups (fasted and non-fasted). Urine volume and osmolality are measured every hour for 4 hours. Test values are recorded as ml of urine excreted

(cumulative), mEg/rat electrolyte excreted, mg/rat urea excreted, and osmolality in milli-Osmoles/Kg $H_2O$. A tolerance test is used to determine significance. $ED_{300}$ is defined as the dose of compound (μg/kg required to lower urine osmolality to 300 m-Osmoles/kg.

TABLE I

| | $K_b$ (pig) | $K_i$ (pig) | $ED_{300}$ (rat) |
|---|---|---|---|
| A. | 12 | 3.9 | 10.1 |
| B. | 170 | - | 73.6 |
| C. | 7.1 | 4.3 | 12.2 |
| D. | 72 | - | 30.8 |

A. [1-(ß-mercapto-ß,ß-cyclopentamethylenepropionic acid)-2-(O-ethyl-D-tyrosine)-4-valine-8-arginine-9-desglycine]vasopressin.

B. [1-(ß-mercapto-ß,ß-cyclopentamethylenepropionic acid)-2-(O-ethyl-D-tyrosine)-4-valine-6-D-cysteine-8-arginine-9-desglycine]vasopressin.

C. [1,6-(6,6-cyclopentamethylene-2-amino-L-suberic acid)-2-(O-ethyl-D-tyrosine)-4-valine-8-arginine -9-desglycine]-vasopressin.

D. [1,6-(6,6-cyclopentamethylene-2-amino-D-suberic acid)-2-(O-ethyl-D-tyrosine)-4-valine-8-arginine -9-desglycine]-vasopressin.

The stereochemistry of the PAS unit in compounds C and D is assigned based on the biological activities of the two compounds. The representative data of Table I demonstrate that the $Pas^{1,6}$ compounds possess equivalent or substantially greater biological activity than their respective dithia compounds of the prior art.

The following examples are intended to demonstrate the preparation and use of this invention. All temperatures are in degrees Centigrade. Terms and symbols are those common in the chemical and peptide arts.

## EXAMPLE 1

Synthesis of Spirocycloalkylene-2-aminoalkandioic acids

(A) Methyl 1-(5-Pent-1-enyl)cyclohexane carboxylate

A solution of lithium diisopropylamide was prepared by adding 2.7M n-butyl lithium (hexane) (37.0 ml, 100 mmol) to a solution of diisopropylamine (15.0 ml, 109 mmol) in dry tetrahydrofuran (170 ml) at -78° under argon, then stirring the subsequent mixture at -78° for 20 minutes. Methyl cyclohexanecarboxylate (13.0 ml, 91 mmol) was added to the solution and the reaction mixture was stirred for 30 minutes at -78°. 5-Iodopent-1-ene (18 g, 91.8 mmol) was added, the reaction continued at -78° for 10 minutes and the mixture was then slowly brought to room temperature. The reaction mixture was diluted with water, acidified (pH=2) with 3N hydrochloric acid and extracted with ether. The ether extracts were washed with saturated sodium thiosulfate (aqueous), dried over magnesium sulfate and evaporated at reduced pressure to give 16 g of 1-(5-pent-1-enyl)cyclohexane carboxylic acid, methyl ester.

(B) 1-(O-Tosyl)hydroxymethyl-1-(5-pent-1-ene)cyclohexane

The methyl ester (A) (16 g, 76.0 mmol) was added to a solution of lithium aluminum hydride (4.6 g, 120.9 mmol) in tetrahydrofuran (205 ml) at room temperature and the resulting mixture was heated at reflux for 3 hours. The reaction was cooled to 0°, treated sequentially with water (5 ml), 3N sodium hydroxide solution (5 ml) and water (15 ml) then filtered. The filtrate was dried over magnesium sulfate, filtered and evaporated to give the titled alcohol: $^1$HNMR ($CDCl_3$): 6.17-5.60 (m, 1H), 5.20-4.80 (m, 2H), 3.40 (S, 2H) and 2.43-0.80 (m, 17H).

This alcohol was dissolved in pyridine (89 ml) and treated with p-toluenesulfonyl chloride (27 g, 142.5 mmol) at 0° for 4 hours. The reaction mixture was poured into water and extracted with petroleum ether, petroleum

ether:ethyl ether (1:1) and ethyl ether. The combined organic extracts were washed with 0.5N HCl (aqueous), saturated sodium bicarbonate (aqueous) and water, dried over magnesium sulfate, filtered and evaporated to give 30.0 g of 1-(O-tosyl)hydroxymethyl-1-(5-pent-1-ene) cyclohexane: $^1$HNMR ($CDCl_3$): 7.98-7.20 (m, 4H), 6.03-5.53 (m, 1H), 5.13-4.78 (m, 2H), 3.82 (S, 2H), 2.43 (S, 3H) and 2.23-0.73 (m, 16H).

(C) 1-Cyanomethyl-1-(5-pent-1-ene) cyclohexane

The tosylate (B) (30 g) was dissolved in dimethylsulfoxide (219 ml) with sodium cyanide (13.6 g, 279 mmol). The resulting mixture was heated at 150° for 18 hours. The reaction mixture was then cooled, poured onto saturated ammonium chloride (aqueous) and extracted with petroleum ether. The organic extracts were dried, filtered and evaporated. Purification of the residue using flash chromatography (silica gel; 15% ethyl acetate/hexane) gave 9.9 g of 1-cyanomethyl-1-(5-pent-1-ene)cyclohexane (57% yield from methyl cyclohexanecarboxylate): $^1$HNMR ($CDCl_3$): 6.12-5.57 (m, 1H), 5.20-4.87 (m, 2H), 2.30 (S, 2H), 2.23-1.82 (m, 2H) and 1.50 (brS, 14H).

(D) 1-Cyanomethyl-1-(4-butan-1-alyl)cyclohexane.

The nitrile (C) (4.9 g, 25.6 mmol) was dissolved in methanol (80 ml). The resulting solution was cooled to -78° and treated with ozone [from a Welsbach ozone generator] until residual ozone was left in solution (blue color). The crude ozonide was treated with methyl sulfide (20 ml) at -78° and, then, slowly warmed to room temperature. After 18 hours, the solvent was removed under reduced pressure and the residue was purified by flash chromatography (silica gel, 20% ethyl acetate/hexane) to give 3.1 g (63%) of 1-cyanomethyl-1-(4-butan-1-alyl)-cyclohexane: NMR ($CDCl_3$): 9.80 (brS, 1H), 2.63-2.38 (m, 2H), 2.33 (S, 2H) and 1.47 (brS, 14H).

(E) 2-D,L-Amino-5-(1-carboxymethyl)cyclohexyl-pentanoic acid

The aldehyde (D) (3.1 g, 16 mmol) was dissolved in 60% aqueous ethanol (30 ml), treated with sodium cyanide (860 mg, 17.6 mmol) and ammonium carbonate (4.0 g, 41.6 mmol). The resulting solution was heated at reflux for 18 hours. Excess carbonate was removed by heating the reaction mixture at 90° for 1 hour without a condenser. The remaining solvent was removed at reduced pressure. The crude product was dissolved in hot ethanol, filtered and evaporated under reduced pressure to give the crude hydantoin: MS: $(M+H)^+ = 264$. The unpurified hydantoin was suspended in water with barium hydroxide hexahydrate (12.0 g, 38 mmol) and heated in a sealed bomb at 170° for 96 hours. The reaction mixture was cooled, diluted with water and filtered. The filtrate was passed over a BioRad AG 50W-X8 ion exchange column and the desired product was eluted off the column with 1N ammonium hydroxide (aqueous). Evaporation of the ammonium hydroxide eluent gave 1.1 g of the titled amino acid (E): MS: $(M+H)^+ = 258$. This compound is also trivially named 6,6-cyclopentamethylene-2-aminosuberic acid (Pas).

(F) 2-D,L-Boc-amino-5-(1-carboxymethyl)cyclohexyl-pentanoic acid

The amino acid (E) was dissolved in 1N sodium hydroxide solution (45 ml) and tert-butanol (45 ml) and the mixture was treated at room temperature with di-tert-butyldicarbonate (980 mg, 4.5 mmol) for 48 hours. The reaction mixture was washed with hexane, acidified with sodium bisulfate and extracted with ethyl acetate. The combined organic extracts were dried, filtered and evaporated to give 1.5 g of the titled compound.

(G) Methyl 2-D,L-boc-amino-5-(1-carbomethoxymethyl) cyclo-hexylpentanoate

The boc-diacid (F) (1.5 g) was dissolved in methylene chloride and treated with excess ethereal diazomethane at 0°. After quenching the excess diazomethane with acetic acid, the solution was evaporated at reduced pressure. The crude ester was purified by flash chromatography (silica gel, 20% ethyl acetate/hexane) to give 900 mg (56%) of the titled product: $^1$HNMR ($CDCl_3$): 5.23-4.87 (m, 1H), 4.50-4.10 (m, 1H), 3.77 (S, 3H), 3.67 (S, 3H), 2.30 (S, 2H), 1.47 (S, 9H) and 1.42 (brS, 16H).

(H) 2-D,L-Boc-amino-5-(1-carbomethoxymethyl)cyclohexylpentanoic acid.

The boc-diester (G) (1.135 g, 2.94 mmol) was dissolved in dioxane (16 ml) and the solution treated at 5° with 3.2 ml 1N NaOH (aqueous). The reaction mixture was immediately warmed to room temperature and stirred for 4 hours. The reaction mixture was acidified (pH = 2) with 3N hydrochloric acid, then evaporated at reduced pressure. The residue was diluted with water and extracted with ethyl acetate. The combined organic extracts were dried, filtered and evaporated. The residue was purified by flash chromatography (silica gel, 5 to 10% methanol/chloroform) to give 836 mg (77%) of titled product. MS: $(M+H)^+ = 372$; $^1$HNMR ($CDCl_3$): 8.02 (brS, 1H), 5.28-5.00 (m, 1H), 4.47-4.07 (m, 1H), 3.65 (S, 3H), 2.30 (S, 2H), 1.47 (S, 9H) and 2.00-1.23 (m, 16H).

(I) Benzyl 2-D,L-boc-amino-5-(1-carbobenzoxymethyl, cyclohexylpentanoate

Boc-diacid (F) (1.34 g, 3.75 mmol) in methylene chloride (30 ml) was treated with benzyl alcohol (1.94 ml, 18.75 mmol), dimethylaminopyridine (1.01 g, 8.25 mmol) and N,N'-dicyclohexylcarbodiimide (1.70 g, 8.25 mmol) at room temperature and the mixture was stirred at room temperature for 72 hours. The reaction mixture was then filtered, evaporated at reduced pressure and the residue was dissolved in ethyl acetate which was washed with 3N hydrochloric acid,

dried over $MgSO_4$ and evaporated at reduced pressure. Purification of the residue by flash chromatography (silica gel, 15% ethyl acetate/hexane) gave 1.27 g (63%) of (I): $^1$HNMR ($CDCl_3$): 7.35 (S, 10H), 5.15 (d,J = 2.4 Hz, 2H), 5.07 (S, 2H) 5.05-4.70 (m, 1H), 4.50-4.05 (m, 1H), 2.27 (S, 2H), 1.45 (S, 9H) and 2.00-1.03 (m, 16H).

(J) 2-Boc-D,L-amino-5-(1-carbobenzoxymethyl)cyclohexylpentanoic acid

The boc-diester (I) (1.27 g, 2.36 mmol) was dissolved in dioxane (15 ml) and treated with 2.6 ml 1N sodium hydroxide solution at room temperature under argon for 4 hours. The reaction mixture was acidified (pH = 2) with 3N hydrochloric acid and evaporated at reduced pressure. The residue was dissolved in ethyl acetate, washed with 3N hydrochloric acid, dried over $MgSO_4$ and evaporated at reduced pressure. The residue was purified by flash chromatography (silica gel, 5% methanol/chloroform) to give 1.03 g of the title product: MS: $MH^+$ = 448); $^1$HNMR ($CDCl_3$): 7.33 (S, 5H), 5.07 (S, 2H), 5.20-4.80 (m, 1H), 4.42-4.05 (m, 1H), 2.32 (S, 2H), 1.43 (S, 9H) and 2.08-1.07 (m, 16H).

(K) Methyl 2-D,L-boc-amino-5-(1-carboxymethyl)cyclohexylpentanoate

The boc-benzyl ester (J) (4 mmol) is dissolved in methylene chloride and treated with excess ethereal diazomethane at room temperature. The residual diazomethane is quenched with acetic acid and the solution is evaporated at reduced pressure. The crude diester is dissolved in methanol and hydrogenated over 10% palladium-on-carbon to give, after flash chromatography (silica gel), the titled compound.

(L) General

Running the above sequence of reactions with methylcyclopentanecarboxylate gives 2-D,L-amino-5-(1-carboxy-methyl)-cyclopentylpentanoic acid and

2-D,L-boc-amino-5-(1-carbobenzoxymethyl) cyclopentyl-pentanoic acid. Using 4-iodobut-1-ene or 6-iodohex-1-ene in place of 5-iodopent-1-ene in the first step of the reaction sequence described in detail above gives 2-D,L-amino-4-(1-carboxymethyl)cyclohexylbutanoic acid, 2-D,L-amino-6-(1-carboxymethyl)cyclohexyl-hexanoic acid as well as their N-boc and 1-benzyl ester derivatives. One skilled in the art will recognize the latter two amino acids are the 2-D,L-amino-spirocyclopentamethylene pimelic and azelaic acids.

## EXAMPLE 2

Synthesis of Linear Peptide Intermediates

(A) 2-Boc-amino-5-(1-carbobenzoxymethyl)cyclohexylpentanoylprolyl-(N-Tos)arginyl-BHA resin.

0.5 Mmoles (0.96 g) of BHA-resin was swollen in 25 ml of methylene chloride in a 30 ml reaction vessel in a manual peptide synthesizer for 4 hours. It was, then, sequentially coupled with protected arginine and proline as well as 2-D,L-boc-amino-5-(1-carbobenzoxymethyl)cyclohexylpentanoic acid using DCC and HBT or DMAP (4-dimethylaminopyridine) as coupling reagent in mixture of dimethylformamide and methylene chloride as solvent.

The amounts and mole ratio of each reagent used in this experiment are as follows:

| A.A. | | Coupling Agent | Catalyst | |
|---|---|---|---|---|
| Boc-Arg(Tos) | 642 mg | DCC 5 ml/0.3M sol. | HBT | 405 mg |
| Boc-Pro | 390 mg | DCC 5 ml/0.3M | HBT | 405 mg |
| Boc-D,L-Pas(Bzl) | 900 mg | DCC 5 ml/0.3M | DMAP | 183 mg |

After the sequential coupling cycles, the peptide resin (A) was treated with acetic anhydride for 1 hour (in order to acetylate any unreacted amino group).

(B) Boc-(O-ethyl)-D-tyrosyl)-phenylalanyl-valyl-asparagyl-(OBzl)-6,6-cyclopentamethylene-2-D,L-aminosuberic acid-prolyl-(N-Tos)arginyl-BHA resin.

The tri-unit resin (A) from above was further sequentially coupled in four cycles with Boc-Asn, Boc-Val, Boc-Phe and Boc-D-Tyr(OEt) using DCC/HBT as coupling reagents and $CH_2Cl_2$/DMF as solvent in a manual peptide synthesizer. Each coupling was monitored by a qualitative ninhydrin test in order to test for completion. Repeated coupling is carried out when the test is positive.

The amounts of reagents used are as follows:

| | A.A. | DCC | HBt |
|---|---|---|---|
| Boc-Asn | 560 mg | 496 mg | 621 mg |
| Boc-Val | 521 mg | 8 ml/0.3M Solution | 621 mg |
| Boc-Phe | 636 mg | 496 mg | 621 mg |
| Boc-D-Tyr(Et) | 672 mg | 496 mg | 621 mg |

The peptide resin was, then, dried in vacuo to give the titled intermediate.

(C) $H_2N$-D-Tyr(Et)-Phe-Val-Asn-D,L-Pas-Pro-$ArgNH_2$

The peptide resin (B) was treated with 20 ml of anhydrous hydrogen fluoride at 0° with 1.5 ml of anisole added as scavanger for 1 hour for the cleavage of the peptide from the resin support and concurrently the deprotection of amino and carboxy groups.

The HF was evaporated under reduced pressure at 0°. The residue was triturated with ether, which was discarded, and was thoroughly extracted with 10 ml x 3 of dimethylformamide and 10 ml x 3 of 30% aqueous acetic acid. The extracts were combined and evaporated under reduced pressure to give an oil which was taken up in 5% aqueous acetic acid and lyophilized to give 195 mg of white powder.

This crude linear peptide, $H_2N$-D-Tyr(Et)-Phe-Val-Asn-D,L-Pas-Pro-$ArgNH_2$ as the acetate salt, was shown to be a mixture of two major components in both HPLC (Ultrasphere ODS® column, 50% aqueous acetonitrile

with 0.1% TFA) and tlc (silica/n-BuOH/ethyl acetate/acetic acid/water 1:1:1:1) with traces of other components.

This crude peptide was purified by counter current distribution (CCD) in BAW (butanol/acetic acid/water) 4:1:5 in 240 transfers. Fractions were checked by tlc and appropriate fractions were pooled, evaporated under reduced pressure and lyophilized from 1% aqueous acetic acid to yield:

| | | |
|---|---|---|
| Fractions | (I) | 28.5 mg |
| Fractions | (II) | 110 mg |
| Fractions | (III) | 35 mg |

Isocratic HPLC (Ultrasphere ODS®; 40% aqueous acetonitrile with 0.1% TFA; flow rate equal to 1.5 ml/min) showed two peaks with retention times of 5.09 and 7.80 minutes respectively. Fraction I contained predominately the 5.09 min. isomer (approximately 90%), fraction II contained an aqual amount of both isomers and fraction III contained mostly the 7.80 min. isomer. FAB-Mass spectrum confirmed the presence of the desired peptide ($MH^+$ = 1061; $MH^-$ = 1059) in all three fractions.

These results indicate the two peaks are the two desired D- and L-isomers of titled linear peptide intermediate (C).

## EXAMPLE 3

D-Tyr(Et)-Phe-Val-Asn-$Pas^{1,6}$-Pro-$ArgNH_2$

The linear peptide (C) from Example 2 (50 mg) was treated with 1N HCl in glacial acetic acid and, then, evaporated in vacuum to dryness. This was repeated three times. The residue was taken up in dried dimethylformamide and evaporated *in vacuo*. This was repeated three times. (This process is to convert the acetic acid salt at the amino and guanidino groups to the corresponding stronger hydrochloric acid salt *in situ* and, thus, eliminate the side reaction of acetylation of the α-amino

group of the D-Tyr by the liberation of the acetic acid. This conversion can also be accomplished by ion exchange column chromatography with some loss of material.)

The treated residue was dissolved in 7.5 ml of dried dimethylformamide, then 12.5 µl of triethylamine was added with stirring, followed by addition of 20 µl of diphenylphosphoryl azide (DPPA). The resulted mixture was stirred under an argon atmosphere at room temperature overnight. The completion of the cyclization is monitored by isocratic HPLC or by tlc.

The reaction mixture was evaporated to dryness and redissolved in 30% acetic acid. The salt was removed by a G-15 Sephadex® gel filtration column. The product was further purified and separated into D- and L-isomers by preparative HPLC in $C_{18}$ column using 45% acetonitrile in water with 0.1% trifluoroacetic acid as mobile phase to give 8.5 mg of compound C of Table 1 above and 5 mg of compound D.

Compound C:

FAB-Mass Spec $(M+H)^+ = 1043$, $(M-H)^- = 1041$

HPLC single peak retention time = 9.72 min.

Amino acid analysis:

| | | | |
|---|---|---|---|
| Aspartic | 1.00 | Tyrosine | 0.97 |
| Proline | 0.69 | Phenylanaline | 0.98 |
| Valine | 0.95 | Arginine | 1.10 |

Compound D:

FAB-Mass Spec $(M+H)^+ = 1043$, $(M-H)^- = 1041$

HPLC single peak retention time = 16.24 min.

Amino acid analysis:

| | | | |
|---|---|---|---|
| Psp | 1.00 | Tyr | 0.92 |
| Pro | 0.76 | Phe | 0.97 |
| Val | 0.77 | Arg | 1.22 |

EXAMPLE 4

D-Tyr(Et)-Phe-Val-Asn-Pas-Arg-Arg$(NH_2)$

The protected peptide intermediate resin, D-Tyr(Et)-Phe-Val-Asn-Pas(Bzl)-Arg(Tos)-Arg(Tos)-BHA is synthesized on 1.0 mmol of benzhydrylamine resin as above. The HF cleavage and cyclization with DPPA are performed as described to give the partially purified peptide.

EXAMPLE 5

Procedure for Terminal Coupling

(A) D-Tyr(Et)-Phe-Val-Asn-Pas-$OCH_3$

The protected peptide intermediate resin, i.e., Boc-D-Tyr(Et)-Phe-Val-Asn-Pas-$OCH_2$-$C_6H_4$-Resin-$OCH_3$ is synthesized manually on a shaker. Boc-Pas$(OCH_2C_6H_4$-Resin)$OCH_3$ is prepared from Pas-$OCH_3$ cesium salt and chloromethyl resin or direct coupling to hydroxymethyl resin with DMAP/DCC in DMF with substitution of 0.35 meq./g. The protected peptide-resin intermediate is synthesized stepwise by deprotecting the Boc-group using 1:1 TFA:$CH_2Cl_2$ and coupling sequentially with the appropriate Boc-amino acids using DCC/HBT as activating and catalyzing reagents on 1.0 mmol scale to give the resin coupled intermediate. The titled peptide is obtained by cleavage from the resin using HF 30 ml in the presence of 3.0 ml of anisole at 0° for 60 min. After evaporation <u>in vacuo</u> to dryness, the residue is washed with anhydrous diethyl ether and is extracted with degassed dimethylformamide, and 40% acetic acid. The extracts were combined, evaporated <u>in vacuo</u> to dryness, taken up in 10% aqueous acetic acid and lyophilized to give the crude linear peptide. Cyclization of this peptide, $NH-_2$-D-Tyr(Et)-Phe-Val-Asn-Pas-$OCH_3$, using DPPA as described above gives the methyl ester of titled compound.

(B) D-Tyr(Et)-Phe-Val-Asn-Pas-OH

A suspension of 0.1 mmol of the methyl ester (A) above in 1:1 dioxane-10% $K_2CO_3$ (aqueous) is stirred at room temperature for 8 hours. The reaction mixture is diluted with water and acidified to pH = 4 with glacial acetic acid. The resulted solution is evaporated *in vacuo* and the residue purified by gel chromatography.

(C) D-Tyr(Et)-Phe-Val-Asn-Pas-$NHNH_2$

The methyl ester (A) above (100 mg) is treated with anhydrous hydrazine in methanol at room temperature to give after evaporation the titled compound, the hydrazide.

(D) Condensation

The 6-Pas acid (20 mg) from B above is reacted with one equivalent of $N^{\varepsilon}$-BocLys-Arg($NH_2$) 2HCl in the presence of DCC, HBT and one equivalent of triethylamine in dimethylformamide followed by treatment with trifluoroacetic acid to produce D-Tyr(Et)-Phe-Val-Asn-$Pas^{1,6}$-Lys-Arg($NH_2$).

Similarly the corresponding 7-Arg acid cyclized peptide and the 7-Pro cyclized peptide acid congeners are made from reacting the acylazide derived from the hydrazide (C) using arginine or proline. Either acid compound is isolated as the potassium salt if desired. One equivalent of Arg-$NH_2$ 2HCl is coupled with the corresponding 7-D-Pro acid made as described using DCC/HBT to give the 7-D-Pro-Arg-$NH_2$.

EXAMPLE 6

D-Tyr(Et)-Phe-Ile-Asn-Pas-MeArgArg$NH_2$

The protected peptide intermediate resin, Boc-D-Tyr-(Et)-Phe-Val-Asn-Pas(Bzl)-N-MeArg(Tos)-Arg(Tos)-BHA is prepared by the solid-phase method on benzhydrylamine resin. On a shaker, 0.5 mmol of BHA resin is used, all amino acids are protected as *tert*.-butyloxycarbonyl on the nitrogen and coupled sequentially using DCC/HBT, while the Pas(Bzl) is coupled using DMAP. The peptide is

cleaved from the resin with deprotection of the side-chain protecting groups using anhydrous HF (20 ml) and anisole (2 ml) at 0° for 60 minutes. The peptide is extracted from the resin with aqueous HOAc/DMF. After evaporation in vacuo to dryness, the peptide residue is washed with anhydrous diethyl ether and cyclized as described with DPPA to give the titled compound.

EXAMPLE 7

(A) Boc-D-Tyr(Et)-Phe(4'-Et)-Val-Asn-Pas(Bzl)-Pro-Arg-(Tos)-Gly-BHA-resin.

(3 mmol) Boc-Val-Asn-Pas(Bzl)-Pro-Arg(Tos)-Gly-BHA-resin is prepared as described above. Further coupling sequentially 1 mmole of this peptide resin with Boc-Phe-(4'-Et) and Boc-D-Tyr(Et) using 3 mmoles of amino acid and 3 mmoles of DCC and 6 mmoles of 1-hydroxybenzotriazole as catalyst. The final peptide resin is washed with methylene chloride and vacuum dried to give the titled product.

(B) D-Tyr(Et)-Phe(4'-Et)-Val-Asn-Pas-Pro-Arg-GlyNH$_2$

1.55 Grams of Boc-D-Tyr(Et)-Phe(4'-Et)-Val-Asn-Pas(Bzl)-Pro-Arg(Tos)-Gly-BHA-resin from A is treated with hydrogen fluoride and subsequent cyclization with an excess of DPPA as described above to give the titled compound.

EXAMPLE 8

D-Tyr(Et)-Phe-Val-Asn-Pas-Pro-Cad

To a solution of the Pro$^7$-acid, prepared as described in Example 5, (0.033 mmol) and mono-Boc-1,5-diaminopentane (20.2 mg, 0.0996 mmol) in dimethylformamide (400 ul), dicyclohexylcarbodiimide (10.3 mg, 0.05 mmol) and 1-hydroxybenzotriazole hydrate (13.4 mg, 0.1 mmol) are added. The reaction mixture is stirred at room temperature for 19 hours. The dimethylformamide is, then, removed under vacuum. The residue is treated with trifluoroacetic acid at 0° for 2 hours. After this time,

the trifluoroacetic acid is removed under vacuum and the residue in 1% acetic acid is passed over a BioRex 70 ($H^+$) ion exchange column. The basic products are washed off the ion exchange column with pyridine buffer ($H_2O$/pyridine/HOAc, 66:30:4) and evaporated. Final purification by preparative HPLC (5µ Ultrasphere ODS®) gives the title compound as the base or the acetate salt.

EXAMPLE 9

Using the methods of synthesis described in detail above, the following specific compounds are produced.

A. [1,6-(6,6-cyclopentamethylene-2-aminosuberic acid)-2-(O-ethyl-D-tyrosine)-4-valine-7-desproline-8-arginine]-vasopressin;

B. [1,6-(6,6-cyclopentamethylene-2-aminosuberic acid)-2-D-valine-3-(O-ethyltyrosine)-4-valine-8-arginine]-vasopressin.

C. [1,6-(6,6-cyclopentamethylene-2-aminosuberic acid)-2-D-cyclohexylalanine-7-desproline-8-arginine]vasopressin.

D. [1,6-(6,6-cyclopentamethylene-2-aminosuberic acid)-2-methionine-4-alanine-7-D-(N-methylalanine)-8-arginine-9-desglycine]vasopressin.

E. [1,6-(6,6-cyclopentamethylene-2-aminosuberic acid)-2-(O-ethyl-D-tyrosine)-4-valine-7-desproline-8-arginine-9-desglycine]vasopressin.

F. [1,6-(6,6-cyclotetramethylene-2-aminosuberic acid)-2-norleucine-3-isoleucine-4-cyclohexylglycine-8-arginine]-vasopressin.

G. [1,6-(6,6-cyclopentamethylene-2-aminosuberic acid)-2-(O-methyltyrosine)-4-valine-7-desproline-8,9-bis-homoarginine]vasopressin.

H. [1,6-(6,6-cyclopentamethylene-2-aminosuberic acid-2-(O-ethyl-D-tyrosine)-4-valine-7-desproline-8-arginine]-vasopressin;

I. [1,6-(6,6-cyclopentamethylene-2-aminosuberic acid)-2-L-tyrosine)-4-valine-7-desproline]vasopressin.

EXAMPLE 10

Parenteral Dosage Unit Compositions:

A preparation which contains 0.1 mg of a peptide of Example 3 as a sterile dry powder for parenteral injection is prepared as follows: 0.5 mg of peptide is dissolved in 1 ml of an aqueous solution of 20 mg of mannitol. The solution is filtered under sterile conditions into a 2 ml ampoule and lyophilized. The reconstituted solution is administered to a patient in need of vasopressin $V_2$-antagonist treatment as necessary, from 1-5 times daily by injection, or in an equivalent continuous i.v. drip injection.

Nasal Dosage Unit Compositions

2.5 Mg of a finely ground peptide of this invention, such as a product of Example 3, is suspended in a mixture of 75 mg of benzyl alcohol and 1.395 g of a suspending agent such as a commercial mixture of semisynthetic glycerides of higher fatty acids. The suspension is placed in an aerosol 10 ml container which is closed with a metering valve and charged with aerosol propellants. The contents comprise 100 unit doses which are administered intranasally to a subject in need of aquaretic therapy from 1-6 times a day.

CLAIMS for BE, CH, DE, FR, GB, IT, LI, LU, NE and SE

1. A chemical compound having the formula:

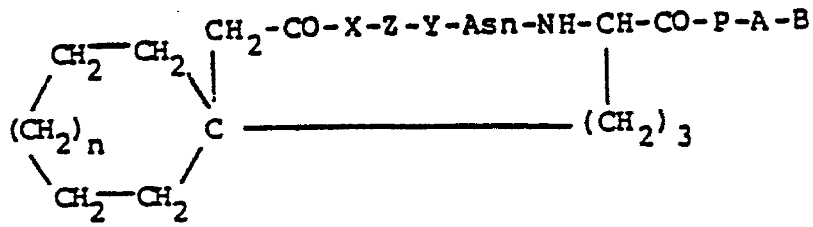

in which:

n is 0 or 1;

P is a single bond or a D- or L-isomer of Pro, Δ-Pro, Ala, MeAla, Arg, Lys, HArg, MeArg, MeLys or MeHArg;

A is a D or L-isomer of Arg, Lys, HArg, MeArg, MeLys, MeHArg or, when B is $-NH-(CH_2)n^1-NH_2$, a single bond;

B is OH, $NH_2$, NHAlk, Gly($NH_2$), Gly(NHAlk) or, when A is a single bond, $-NH-(CH_2)n^1-NH_2$;

Z is Phe, Phe(4'-Alk), Tyr(Alk), Ile or Tyr;

X is a D- or L-isomer of Phe, Phe(4'-Alk), Val, Nva, Leu, Ile, Pba, Nle, Cha, Abu, Met, Chg, Tyr, Trp or Tyr(Alk); and

Y is Val, Ile, Abu, Ala, Chg, Gln, Lys, Cha, Nle, Thr, Phe, Leu or Gly, and $n^1$ is 2 to 6; or a pharmaceutically acceptable salt, optical isomer or ester prodrug thereof.

2. The compounds according to claim 1 in which X is a D-isomer.

3. The compounds according to claim 1 in which X is a D-Tyr(Alk) and Y is Val.

4. The compounds according to claim 1 in which X is D-Tyr(Alk), Y is Val, n is 1 and P is a single bond.

5. The compounds according to claim 1 in which X is D-Tyr(Et), Y is Val, n is 1 and B is $NH_2$.

6. The compound according to claim 1 in which the compound is [1,6-(6,6-cyclopentamethylene-2-amino-D-suberic acid)-2-(O-ethyl-D-tyrosine)-4-valine-9-desglycine-8-arginine]vasopressin.

7. The compound according to claim 1 in which the compound is [1,6-(6,6-cyclopentamethylene-2-amino-L-suberic acid)-2-(O-ethyl-D-tyrosine)-4-valine-9-desglycine-8-arginine]vasopressin.

8. The compound according to claim 1 in which the compound is [1,6-(6,6-cyclopentamethylene-2-amino-D-suberic acid)-2-(O-ethyl-D-tyrosine)-4-valine-7-desproline-8-arginine-9-arginin-amide]vasopressin.

9. The compound according to claim 1 in which the compound is [1,6-(6,6-cyclopentamethylene-2-amino-D-suberic acid)-2-(O-ethyl-D-tyrosine)-4-valine-7-D-proline-9-desglycine]vasopressin.

10. A pharmaceutical composition comprising a compound as defined in any of claims 1 to 9 and a pharmaceutically acceptable carrier.

11. A compound according to any one of claims 1 to 9 for use as a vasopressin antagonist.

12. A compound according to any one of claims 1 to 9 for use as a water diuretic.

13. A compound according to any one of claims 1 to 9 for use as an anti-hypertensive.

14. A chemical compound having the formula:

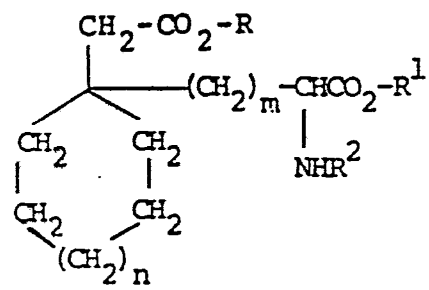

in which:

m is an integer of from 2-4;

n is 0 or 1;

R and $R^1$ are, each, hydrogen or a carboxy protecting group; and

$R^2$ is hydrogen or an amino protecting group.

15. The compound according to claim 14 in which n is 1 and R, $R^1$ and $R^2$ are each hydrogen.

16. The compound according to claim 14 in which n is 1, R is hydrogen or benzyl, $R^1$ is hydrogen and $R^2$ is Boc.

17. The compound according to claim 14 in which n is 1, R and $R^1$ are hydrogen and $R^2$ is Boc.

18. A chemical compound having the formula:

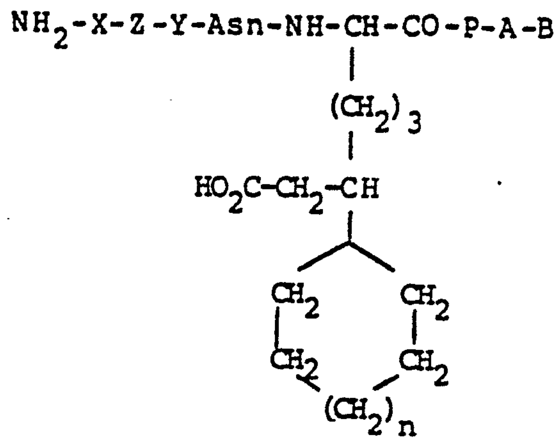

in which:

n is 0 or 1;

P is a single bond or a D- or L-isomer of Pro, Δ-Pro, Ala, MeAla, Arg, Lys, HArg, MeArg, MeLys or MeHArg;

A is a D or L-isomer of Arg, Lys, HArg, MeArg, MeLys, MeHArg or, when B is $-NH-(CH_2)n^1-NH_2$ a single bond;

B is OH, $NH_2$, NHAlk, Gly($NH_2$), Gly(NHAlk) or, when A is a single bond, $-NH-(CH_2)n^1-NH_2$;

Z is Phe, Phe(4'-Alk), Tyr(Alk), Ile or Tyr;

X is a D- or L-isomer of Phe, Phe(4'-Alk), Val, Nva, Leu, Ile, Pba, Nle, Cha, Abu, Met, Chg, Tyr, Tyr(Alk) or, Trp;

Y is Val, Ile, Abu, Ala, Chg, Gln, Lys, Cha, Nle, Thr, Phe, Leu or Gly, and $n^1$ is 2 to 6 or an acid addition salt thereof.

19. The chemical compound according to claim 18 in which n is 1, P is a single bond, A is Arg, B is $NH_2$, Z is Phe, X is D-Tyr(Et) and Y is Val.

20. The chemical compound according to claim 18 in which n is 1, P is Pro, A is Arg, B is $NH_2$, Z is Phe, X is D-Tyr(Et) and Y is Val.

21. The chemical compound according to claim 18 in which n is 1, P is Pro, A is Arg, B is Gly($NH_2$), Z is Phe, X is D-Tyr(Et) and Y is Val.

22. A process for preparing a compound having the formula:

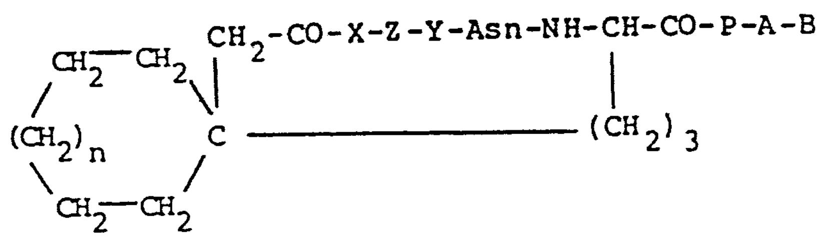

in which:

n is 0 or 1;

P is a single bond or a D- or L-isomer of Pro, Δ-Pro, Ala, MeAla, Arg, Lys, HArg, MeArg, MeLys or MeHArg;

A is a D or L-isomer of Arg, Lys, HArg, MeArg, MeLys, MeHArg or, when B is $-NH-(CH_2)n^1-NH_2$, a single bond;

B is OH, $NH_2$, NHAlk, Gly($NH_2$), Gly(NHAlk) or, when A is a single bond, $-NH-(CH_2)n^1-NH_2$;

Z is Phe, Phe(4'-Alk), Tyr(Alk), Ile or Tyr;

X is a D- or L-isomer of Phe, Phe(4'-Alk), Val, Nva, Leu, Ile, Pba, Nle, Cha, Abu, Met, Chg, Tyr, Trp or Tyr(Alk);

Y is Val, Ile, Abu, Ala, Chg, Gln, Lys, Cha, Nle, Thr, Phe, Leu or Gly, and $n^1$ is 2 to 6; or a pharmaceutically acceptable salt, optical isomer or ester prodrug thereof,

which comprises cyclizing an optionally protected compound of the formula:

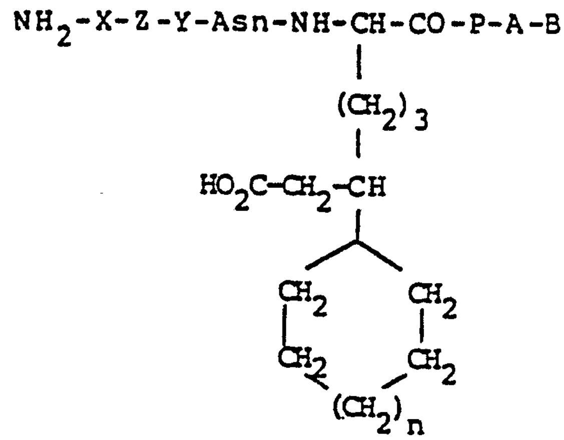

in which X, Z, Y, P, A, B and n are as defined above; and optionally thereafter in any order,

(a) removing any protecting groups;

(b) reacting the Pas(OH) or P(OH) compounds, whenever present, with A as defined above, either in acid protected or amide form; and

(c) forming a pharmaceutically acceptable salt thereof.

23. A process for preparing a compound having the formula:

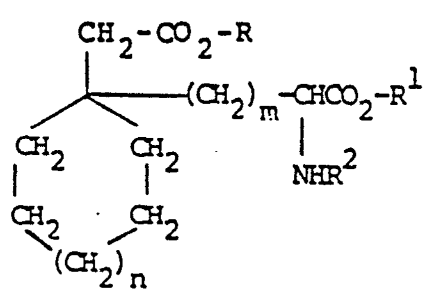

in which:

m is an integer of from 2-4;

n is 0 or 1;

R and $R^1$ are, each, hydrogen or a carboxy protecting group; and

$R^2$ is hydrogen or an amino protecting group, which comprises inserting a terminally unsaturated hydrocarbon chain at the spiro carbon of a cycloalkane carboxylate; homologizing the carboxylate; functionalizing the unsaturated center of the side chain; and preparing an amino acid therefrom.

CLAIMS for AT, GR and ES

1. A process for preparing a compound having the formula:

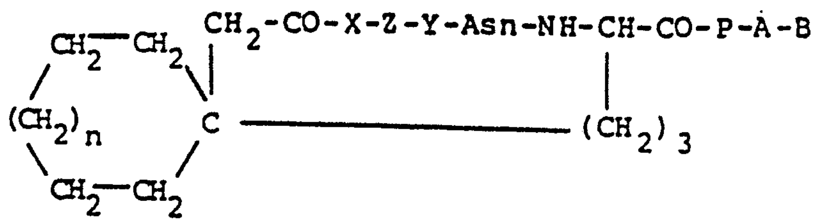

in which:

n is 0 or 1;

P is a single bond or a D- or L-isomer of Pro, Δ-Pro, Ala, MeAla, Arg, Lys, HArg, MeArg, MeLys or MeHArg;

A is a D or L-isomer of Arg, Lys, HArg, MeArg, MeLys, MeHArg or, when B is $-NH-(CH_2)n^1-NH_2$, a single bond;

B is OH, $NH_2$, NHAlk, Gly($NH_2$), Gly(NHAlk) or, when A is a single bond, $-NH-(CH_2)n^1-NH_2$;

Z is Phe, Phe(4'-Alk), Tyr(Alk), Ile or Tyr;

X is a D- or L-isomer of Phe, Phe(4'-Alk), Val, Nva, Leu, Ile, Pba, Nle, Cha, Abu, Met, Chg, Tyr, Trp or Tyr(Alk);

Y is Val, Ile, Abu, Ala, Chg, Gln, Lys, Cha, Nle, Thr, Phe, Leu or Gly, and $n^1$ is 2 to 6 or a pharmaceutically acceptable salt, optical isomer or ester prodrug thereof,

which comprises cyclizing an optionally protected compound of the formula:

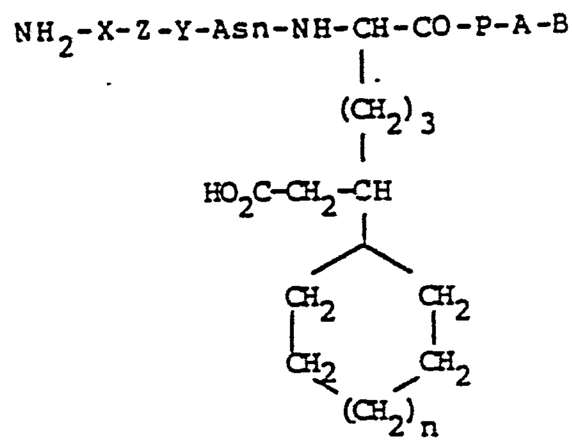

in which X, Z, Y, P, A, B and n are as defined above; and optionally thereafter in any order,

(a) removing any protecting groups;

(b) reacting the Pas(OH) or P(OH) compounds, whenever present, with A as defined above, either in acid protected or amide form; and

(c) forming a pharmaceutically acceptable salt thereof.

2. The process according to claim 1 in which X is a D-isomer.

3. The process according to claim 1 in which X is a D-Tyr(Alk) and Y is Val.

4. The process according to claim 1 in which X is D-Tyr(Alk), Y is Val, n is 1 and P is a single bond.

5. The process according to claim 1 in which X is D-Tyr(Et), Y is Val, n is 1 and B is $NH_2$.

6. A process for preparing a compound having the formula:

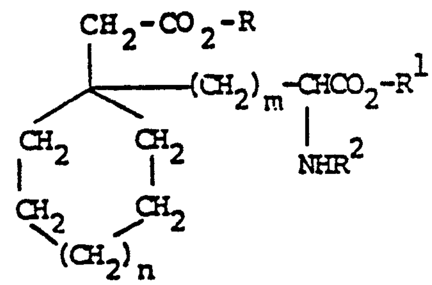

in which:

m is an integer of from 2-4;

n is 0 or 1;

R and $R^1$ are, each, hydrogen or a carboxy protecting group; and

$R^2$ is hydrogen or an amino protecting group, which comprises inserting a terminally unsaturate hydrocarbon chain at the spiro carbon of a cycloalkane carboxylate; homologizing the carboxylate; functionalizing the unsaturated center of the side chain; and preparing an amino acid therefrom.